# EUROPEAN PATENT SPECIFICATION

(11) **EP 3 631 007 B1**
(45) Date of publication and mention of the grant of the patent: **24.03.2021**
(21) Application number: 18728084.7
(22) Date of filing: 25.05.2018
(51) Int. Cl.: C12Q 1/6818

(54) **MULTIPLEX NUCLEIC ACID AMPLIFICATION ASSAY**
MULTIPLEX-NUKLEINSÄUREAMPLIFIKATIONSTEST
DOSAGE PAR AMPLIFICATION MULTIPLEX D'ACIDES NUCLÉIQUES

(30) Priority: 25.05.2017 US 201762511151 P
(43) Date of publication of application: 08.04.2020
(73) Proprietor: Roche Diagnostics GmbH, 68305 Mannheim (DE); F. Hoffmann-La Roche AG, 4070 Basel (CH)
(72) Inventor: WILL, Stephen Gordon, Pleasanton, California 94588 (US)
(74) Representative: Hövelmann, Sascha Alexander
(86) International application number: PCT/EP2018/063751
(87) International publication number: WO 2018/215640

(56) References cited:
- EP-A1- 2 228 454
- WO-A1-96/15270
- WO-A1-2010/068576

## Description

### FIELD OF THE DISCLOSURE

The disclosure relates generally to *in vitro* amplification and detection of nucleic acids. Specifically, the disclosure relates to a single-tube multiplex assay, capable of simultaneously amplifying and detecting multiple nucleic acid targets, using multiple sets of hybridization primers and probes, wherein the probes are labeled with the same reporter label. The assay can be further multiplexed with the use of several reporters.

### BACKGROUND OF THE DISCLOSURE

The polymerase chain reaction (PCR) has become a ubiquitous tool of biomedical research, disease monitoring and diagnostics. Amplification of nucleic acid sequences by PCR is described in U.S. Patent Nos. 4,683,195, 4,683,202, and 4,965,188. PCR is now well known in the art and has been described extensively in the scientific literature. See PCR Applications, ((1999) Innis et al., eds., Academic Press, San Diego), PCR Strategies, ((1995) Innis et al., eds., Academic Press, San Diego); PCR Protocols, ((1990) Innis et al., eds., Academic Press, San Diego), and PCR Technology, ((1989) Erlich, ed., Stockton Press, New York). A "real-time" PCR assay is able to simultaneously amplify and detect and quantify the starting amount of the target sequence. The basic TaqMan real-time PCR assay using nuclease activity of the DNA polymerase is described in Holland et al., (1991) Proc. Natl. Acad. Sci. 88:7276-7280 and U.S. Patent No. 5,210,015. The real-time PCR without the nuclease activity (a nuclease-free assay) has been described in a U.S. application Serial No. 12/330,694 filed on December 9, 2008. The use of fluorescent probes in real-time PCR is described in U.S. Patent No. 5,538,848 Target probe detection methods are further disclosed in EP2228454 A1, WO96/15270 A1 and WO2010/068576 A1.

A typical real-time PCR protocol involves the use of a labeled probe, specific for each target sequence. The probe is preferably labeled with one or more fluorescent moieties, which emit light of a detectable wavelength. Upon hybridizing to the target sequence or its amplicon, the probe exhibits a detectable change in fluorescent emission.

The major challenge of the real-time assay however remains the ability to analyze numerous targets in a single tube. In virtually every field of medicine and diagnostics, the number of loci of interest increases rapidly. For example, multiple loci must be analyzed in forensic DNA profiling, pathogenic microorganism detection, multi-locus genetic disease screening and multi-gene expression studies, to name a few. With the current methods, the ability to multiplex an assay is limited by the detection instruments. Specifically, the use of multiple probes in the same reaction requires the use of distinct fluorescent labels. To simultaneously detect multiple probes, an instrument must be able to discriminate among the light signals emitted by each probe. The current technology does not permit detection of more than four separate wavelengths in the same reaction vessel. For example, Bell et al. ("Real-time quantitative PCR in parasitology," Trends in Parasitol. (2002) 18(8):337-342.) recently surveyed available real-time quantitative PCR thermal cyclers and reported that none have more than four optical detection channels. Therefore, using one uniquely-labeled probe per target, no more than four separate targets can be detected in the same vessel. In practice, at least one target is usually a control nucleic acid. Accordingly, in practice, no more than three experimental targets can be detected in the same tube. Since the optical hardware may offer at most, a small incremental improvement, the ability to multiplex an assay will not keep pace with the clinical needs, unless radical changes in the amplification and detection strategy are made.

### SUMMARY OF THE DISCLOSURE

Provided herein is a method for detection of a plurality of target nucleic acids in a single sample container comprising the steps of: (a) contacting a sample suspected of containing a plurality of target nucleic acids with (i) a first primer/probe set including a first labeled probe and a first primer, and (ii) a second primer/probe set including a second labeled probe and a second primer, wherein the first primer anneals to a target nucleic acid of the plurality of target nucleic acids at a first temperature and the second primer anneals to an additional nucleic acid of the plurality of target nucleic acids at a second temperature and the first temperature is higher than the second temperature; (b) amplifying the plurality of target nucleic acids in the sample in an amplification reaction that includes at least a two-step annealing/elongation temperature profile including a first step comprising the first temperature and a second step comprising the second temperature; (c) detecting, sequentially, a detectable signal from the first and second labels, respectively over at least a portion of the annealing/elongation temperature profile; and (d) measuring a relative amount of each target nucleic acid in the plurality of target nucleic acids. In some embodiments, said first and second labeled probes are each labeled with the same reporter moiety. In certain embodiments, said reporter moiety is fluorescent. In some embodiments, said first and second labeled probes are each labeled with a reporter moiety and a quencher moiety. In certain embodiments, said reporter moiety and said quencher moiety are fluorophores. In some embodiments, said reporter moiety is a fluorophore and said quencher moiety is a dark quencher. In some embodiments, said detecting step further comprises (i) detecting a first signal during said first step which corresponds to signal emitted by said first label, (ii) detecting a second signal during said second step, and (iii) subtracting said first signal from said second signal to identify the detectable signal emitted by said second label. In some embodiments, said first and second steps, respectively, are held for at least 10 seconds. In certain embodiments, said first and second steps, respectively, are held for between 5-10 seconds. Herein, each of the annealing step and/or the extension step can be held at a constant temperature for the indicated period of time. In certain embodiments, each of the annealing step and/or the extension step can be held at a constant temperature for at least 10 seconds, for at least 15 seconds, for at least 20 seconds, or between 5-10 seconds. In some embodiments, amplifying the plurality of target nucleic acids in the sample includes a first step comprising holding the reaction at a constant first temperature for at least 5-10 seconds and subsequently holding the reaction at a constant second temperature for at least 5-10 seconds. In certain embodiments, each of the first and/or the second temperature are held for at least 10 seconds, for at least 15 seconds, for at least 20 seconds, or between 5-10 seconds. In some embodiments, the first and second temperatures are at least 15 degrees apart, particularly, at least 12 degrees apart, and at least about 10 degrees apart. Further, in some embodiments, the first and second labeled probes are each labeled with the same reporter moiety and the detecting step further comprises (i) detecting a first signal during said first step which corresponds to signal emitted by said first label, (ii) detecting a second signal during said second step, and (iii) subtracting said first signal from said second signal to identify the detectable signal emitted by said second label. In some embodiments, said amplification reaction is performed in the presence of a mixture of DNA polymerases.

Also disclosed is a kit for detection of a plurality of target nucleic acids in a single sample container comprising, in separate containers, vials, or compartments, (i) a first primer/probe set including a first labeled probe and a first primer, and (ii) a second primer/probe set including a second labeled probe and a second primer, wherein said first primer anneals to a target nucleic acid of the plurality of target nucleic acids at a first temperature and the second primer anneals to an additional nucleic acid of said plurality of the target nucleic acids at a second temperature and the first temperature is higher than the second temperature. The kit can further include reagents necessary for amplification of target nucleic acids, optionally comprising a control nucleic acid of known concentration. In some embodiments, said first and second labeled probes are each labeled with the same reporter moiety. In certain embodiments, said reporter moiety is fluorescent. In some embodiments, said first and second labeled probes are each labeled with a reporter moiety and a quencher moiety. In certain embodiments, said reporter moiety and said quencher moiety are fluorophores. In certain embodiments, said reporter moiety is a fluorophore and said quencher moiety is a dark quencher. In some embodiments,

the kit further comprises a control nucleic acid of known concentration. In some embodiments, the single sample container comprises a sample processing tubule including a tube defining a fluid flow channel and a plurality of segments positioned therein, wherein at least one segment of the tube comprises said first and second primer/probe sets. Herein, said tube may further comprise one or more additional segments including reagents necessary for amplification of target nucleic acids. In some embodiments, said reagents comprise a mixture of DNA polymerases.

### BRIEF DESCRIPTION OF THE DRAWINGS

Fig. 1 is a graphical representation of the amplification profile of two target sequences in a sample using primer/probe A and primer/probe B using a two-step primer annealing/elongation temperature profile.
Fig. 2 shows the relative fluorescence resulting from the amplification of two target sequences in a sample using two separate sets of primers and probes, primer/probe A and primer/probe B, wherein the amplification process includes a two-step primer annealing/elongation temperature profile.

### DETAILED DESCRIPTION OF THE DISCLOSURE

### Definitions

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The articles "a" and "an" are used herein to refer to one or to more than one (i.e., to at least one) of the grammatical object of the article. By way of example, "an element" means one element or more than one element.

The terms "detect," "detecting," "detection," and similar terms are used in this application to broadly refer to a process or discovering or determining the presence or an absence, as well as a degree, quantity, or level, or probability of occurrence of something. For example, the term "detecting" when used in reference to a target nucleic acid sequence, can denote discovery or determination of the presence, absence, level or quantity, as well as a probability or likelihood of the presence or absence of the sequence. It is to be understood that the expressions "detecting presence or absence," "detection of presence or absence" and related expressions include qualitative and quantitative detection. For example, quantitative detection includes the determination of level, quantity or amounts of HIV-associated nucleic acid sequences in a sample.

The terms "nucleic acid," "polynucleotide," and "oligonucleotide" refer to polymers of nucleotides (e.g., ribonucleotides or deoxyribo-nucleotides) and includes naturally-occurring (adenosine, guanidine, cytosine, uracil and thymidine), non-naturally occurring, and modified nucleic acids. The term is not limited by length (e.g., number of monomers) of the polymer. A nucleic acid may be single-stranded or double-stranded and will generally contain 5'-3' phosphodiester bonds, although in some cases, nucleotide analogs may have other linkages. Monomers are typically referred to as nucleotides. The term "non-natural nucleotide" or "modified nucleotide" refers to a nucleotide that contains a modified nitrogenous base, sugar or phosphate group, or that incorporates a non-natural moiety in its structure. Examples of non-natural nucleotides include dideoxynucleotides, biotinylated, aminated, deaminated, alkylated, benzylated and fluorophor-labeled nucleotides.

The term "primer" refers to a short nucleic acid (an oligonucleotide) that acts as a point of initiation of polynucleotide strand synthesis by a nucleic acid polymerase under suitable conditions. Polynucleotide synthesis and amplification reactions typically include an appropriate buffer, dNTPs and/or rNTPs, and one or more optional cofactors, and are carried out at a suitable temperature. A primer typically includes at least one target-hybridized region that is at least substantially complementary to the target sequence. This region of is typically about 15 to about 40 nucleotides in length. A "primer pair" refers to a forward primer and reverse primer (sometimes called 5' and 3' primers) that are complementary to opposite strands of a target sequence and designed to amplify the target sequence. The forward and reverse primers are arranged within an amplifiable distance of each other on the target sequence, e.g., about 10-5000 nucleotides, or about 25-500 nucleotides.

As used herein, "probe" means any molecule that is capable of selectively binding to a specifically intended target biomolecule, for example, a nucleic acid sequence of interest to be bound, captured or hybridized by the probe.

The words "complementary" or "complementarity" refer to the ability of a nucleic acid in a polynucleotide to form a base pair with another nucleic acid in a second polynucleotide. For example, the sequence 5'-A-G-T-3' (5'-A-G-U-3' for RNA) is complementary to the sequence 3'-T-C-A-5' (3'-U-C-A-5' for RNA). Complementarity may be partial, in which only some of the nucleic acids match according to base pairing, or complete, where all the nucleic acids match according to base pairing. A probe or primer is considered "specific for" a target sequence if it is at least partially complementary to the target sequence. Depending on the conditions, the degree of complementarity to the target sequence is typically higher for a shorter nucleic acid such as a primer (e.g., greater than 80%, 90%, 95%, or higher) than for a longer sequence.

The term "amplification conditions" or similar expressions refer to conditions in a nucleic acid amplification reaction (e.g., PCR amplification) that allow for hybridization and template-dependent extension of the primers. The term "amplicon" refers to a nucleic acid molecule that contains all or a fragment of the target nucleic acid sequence and that is formed as the product of *in vitro* amplification by any suitable amplification method. Various PCR conditions are described in PCR Strategies (Innis et al., 1995, Academic Press, San Diego, CA) at Chapter 14; PCR Protocols: A Guide to Methods and Applications (Innis et al., Academic Press, NY, 1990) The term "thermostable nucleic acid polymerase" or "thermostable polymerase" refers to a polymerase enzyme, which is relatively stable at elevated temperatures when compared, for example, to polymerases from E. coli. A thermostable polymerase is suitable for use under temperature cycling conditions typical of the polymerase chain reaction ("PCR"). Exemplary thermostable polymerases include those from Thermus thermophilus, Thermus caldophilus, Thermus sp. Z05 (see, e.g., U.S. Patent No. 5,674,738) and mutants of the Thermus sp. Z05 polymerase, Thermus aquaticus, Thermus flavus, Thermus filiformis, Thermus sp. sps17, Deinococcus radiodurans, Hot Spring family B/clone 7, Bacillus stearothermophilus, Bacillus caldotenax, Thermotoga maritima, Thermotoga neapolitana and Thermosipho africanus, and modified versions thereof.

The term "sample" or "biological sample" refers to any composition containing or presumed to contain nucleic acid from an individual. The term includes purified or separated components of cells, tissues, or blood, e.g., DNA, RNA, proteins, cell-free portions, or cell lysates. In a specific embodiment, analysis is conducted on whole blood samples. As used herein, a "whole blood sample" includes blood drawn from the body from which no constituent, such as plasma or platelets, has been removed. Generally, the sample is unmodified except for the presence of an anticoagulant. A sample can also refer to other types of biological samples, e.g., plasma, serum, blood components (buffy coat), and dried blood spots. Samples also may include constituents and components of *in vitro* cultures of cells obtained from an individual, including cell lines.

The term "kit" refers to any manufacture (e.g., a package or a container) including at least one device comprising a solid support, as described herein for specifically amplifying, capturing, tagging/converting or detecting a target nucleic acid sequence as described herein. The kit can further include instructions for use, supplemental reagents and/or components or modules used in the method described herein or a step thereof.

A "fluorophore" is a compound or a moiety attached for example, to a nucleic acid, which is capable of emitting light radiation when excited by a light of a suitable wavelength. Typical fluorescent dyes include rhodamine dyes, cyanine dyes, fluorescein dyes and BODIPY® dyes. A fluorophore is a fluorescent chromophore.

"FRET" or "fluorescent resonance energy transfer" or "Foerster resonance energy transfer" is a transfer of energy between at least two chromophores, a donor chromophore and an acceptor chromophore (referred to as a quencher). The donor typically transfers the energy to the acceptor when the donor is excited by light radiation with a suitable wavelength. The acceptor typically re-emits the transferred energy in the form of light radiation with a different wavelength. When the acceptor is a "dark" quencher, it dissipates the transferred energy in a form other than light. Whether a particular fluorophore acts as a donor or an acceptor depends on the properties of the other member of the FRET pair. Commonly used donor-acceptor pairs include the FAM-TAMRA pair. Commonly used quenchers are DABCYL and TAMRA. Commonly used dark quenchers are BlackHole Quenchers™ (BHQ), Biosearch Technologies, Inc. (Novato, Cal.), Iowa Black™, Integrated DNA Tech., Inc. (Coralville, Iowa), BlackBerry™ Quencher 650 (BBQ-650), Berry & Assoc., (Dexter, Mich.). Commonly used donor-quencher pairs include the FAM-BHQ pair.

"Hybridization" is an interaction between two usually single-stranded or at least partially single-stranded nucleic acids. Hybridization occurs as a result of base-pairing between nucleobases and involves physicochemical processes such as hydrogen bonding, solvent exclusion, base stacking and the like. Hybridization can occur between fully-complementary or partially complementary nucleic acid strands. The ability of nucleic acids to hybridize is influenced by temperature and other hybridization conditions, which can be manipulated in order for the hybridization of even partially complementary nucleic acids to occur. Hybridization of nucleic acids is well known in the art and has been extensively described in Ausubel (Eds.) Current Protocols in Molecular Biology, v. I, II and III (1997).

A "label" refers to a moiety attached (covalently or non-covalently), to a molecule, which moiety is capable of providing information about the molecule. Exemplary labels include fluorescent labels, radioactive labels, and mass-modifying groups.

"Annealing temperature" refers to the temperature at which one half of a population of complementary single-stranded nucleic acid molecules hybridize in homoduplexes or heteroduplexes. The annealing temperature can be based on the melting temperature (Tₘ), i.e., the temperature at which one half of a population of double-stranded nucleic acid molecules disassociate. The annealing temperature is affected by ionic strength and pH of the solution, as well as concentration, base composition and secondary structure. The annealing temperature of a pair of complementary nucleic acids under given conditions can be determined experimentally or predicted with the help of commercial software, such as Visual OMP™ (DNA Software, Inc., Ann Arbor, Mich.).

### Methods

The present disclosure provides a method of simultaneous multiplex detection of nucleic acid targets. In one embodiment, the method utilizes multiple probes labeled with the same reporter moiety, together with multiple primers each having a unique annealing temperature with a given target sequence. Because the primers can be identified by their respective annealing temperatures, the same fluorophores can be used to generate a detectable signal for each primer. The assay may be further multiplexed using several sets of primers/probes, each set including a probe labeled with a separate reporter moiety, up to the number of moieties distinguishable by the detection instrument.

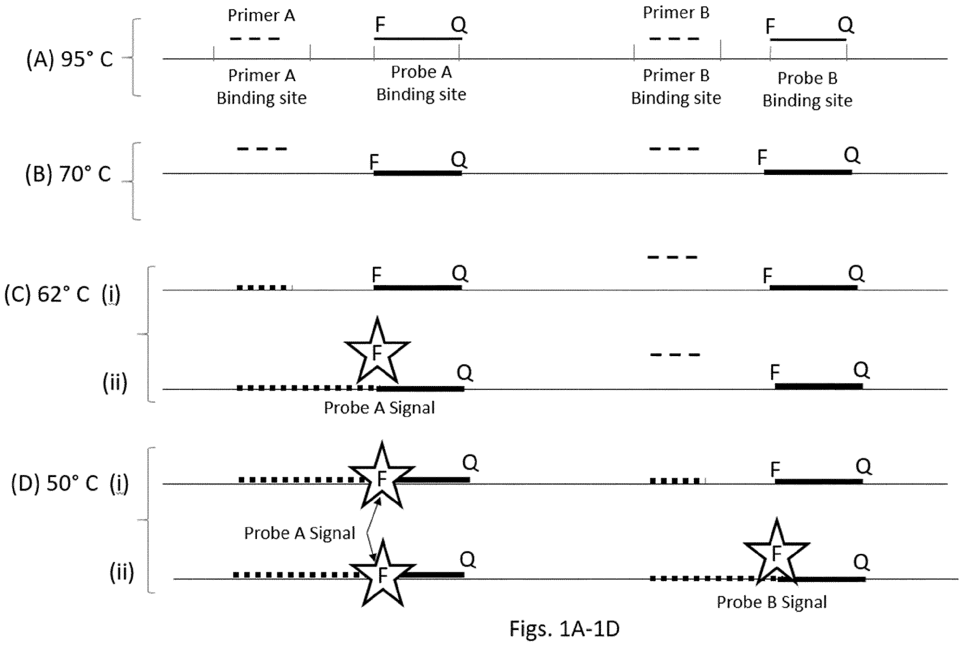

The methods described herein include the detection of signals produced by each primer set during each phase of primer annealing and elongation. The method is illustrated, e.g., in Fig. 1. A sample including a plurality of target nucleic acid sequences is mixed with (i) a first primer/probe set including a first labeled probe and a first primer (Probe A and Primer A in Fig. 1), and (ii) a second primer/probe set including a second labeled probe and a second primer (Probe B and Primer B in Fig. 1), wherein the first primer is designed to anneal to a target nucleic acid in the sample, if present, at a first temperature and the second primer is designed to anneal to an additional nucleic acid in the sample, if present, at a second temperature, and the first temperature is higher than the second temperature. The target nucleic acid sequences in the mixture are denatured, e.g., at 95°C (Panel A), and the temperature is gradually reduced, e.g., to 70°C, to allow the probes to anneal to their respective target sequences (Panel B). The temperature of the mixture is gradually decreased to a first temperature in a multi-step primer annealing/elongation profile, e.g., 62°C (Panel C), to allow Primer A to anneal to the target sequence (Panel C(i)) and elongate (Panel C(ii)). As Primer A is elongated, it hydrolyzes Probe A, generating a detectable signal. At this point in the process, because only Primer A has annealed to the target, the detectable signal is solely attributed to annealing/elongation of Primer A and its respective target sequence.

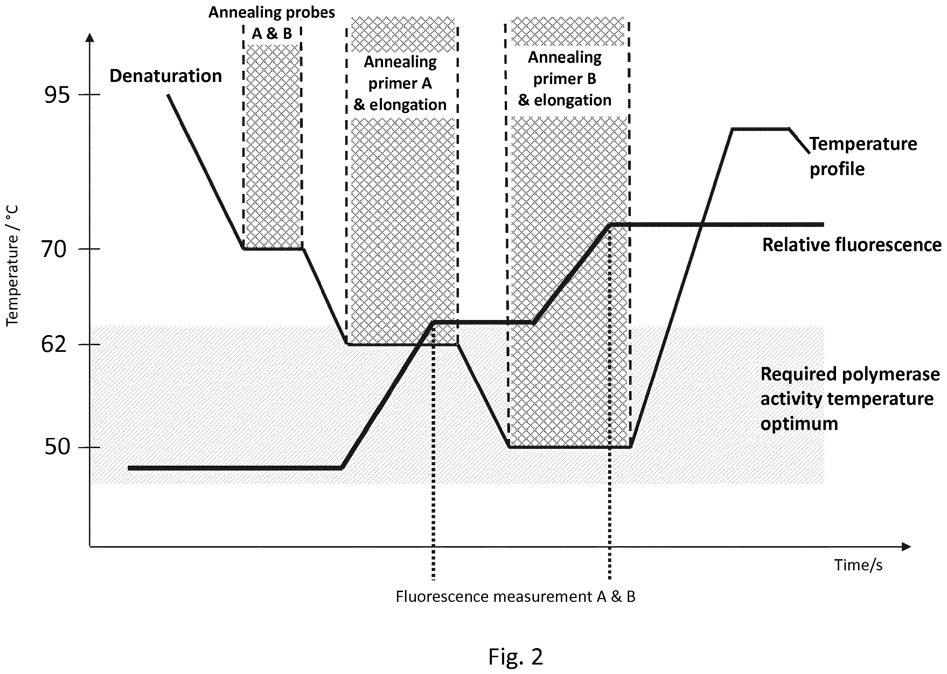

The temperature is then lowered once more to the second temperature of the primer annealing/elongation profile, e.g., 50°C (Panel D), at which point, Primer B anneals with the target sequence (Panel D(i)) and is elongated (Panel D(ii)), eventually hybridizing Probe B and generating a supplemental detectable signal. The detectable signal from the annealing/ elongation of Primer A is not extinguished when the temperature is lowered to the second temperature, and therefore, the cumulative detectable signal at the second temperature corresponds to the signal associated with annealing and elongation of Primer A and Primer B. The detectable signal associated with Primer B can be calculated by subtracting the detectable signal at the second temperature from that observed at the first temperature. The method is also illustrated in Fig. 2 wherein the temperature is plotted against fluorescence. As the temperature is decreased, the relative fluorescence signal increases.

It will be understood that while the method is illustrated in the figures and described above using two primer/probe sets, multiple primer/probe sets can be mixed with the sample simultaneously in order to achieve a higher level of multiplexing, as long as the annealing temperature of each primer and target sequence in an amplification profile is distinct from that of the other primer/target sequences in the sample. In one embodiment, the annealing temperatures of each primer/target sequence are at least 15 degrees apart, particularly, at least 12 degrees apart, and at least about 10 degrees apart.

The probes can be labeled with the same reporter moiety. The reporter moiety can be a chromophore, e.g., a fluorophore, and if two chromophores are used as the reporter moiety, one typically is a reporter chromophore and the other is a quencher. Both chromophores may be fluorophores or one of the chromophores may be a non-fluorescent quencher. Examples of suitable fluorophores include dyes of the fluorescein family (FAM, HEX, TET, JOE, NAN and ZOE), rhodamine family (Texas Red, ROX, R110, R6G and TAMRA), cyanine family (Cy2, Cy3, Cy3.5, Cy5, Cy5.5, and Cy7) coumarin family, oxazine family, thiazine family, squaranine family and other families of fluorescent dyes suitable for the labeling and detection of nucleic acids. The second chromophore may be incorporated into the same probe oligonucleotide or a separate probe oligonucleotide. Commonly used dark quenchers include BlackHole Quenchers™ (BHQ), (Biosearch Technologies, Inc., Novato, Cal.), Iowa Black™, (Integrated DNA Tech., Inc., Coralville, Iowa), and BlackBerry™ Quencher 650 (BBQ-650), (Berry & Assoc., Dexter, Mich.).

In some embodiments, each probe is labeled with two chromophores forming a FRET pair. In some embodiments, both chromophores are fluorophores. In other embodiments one chromophore is a non-fluorescent quencher. The chromophores forming the FRET pair may be conjugated to the same or separate probe molecules. The use of FRET probes in a melting assay has been described in U.S. Patent No. 6,174,670 and in De Silva et al., (1998) "Rapid genotyping and quantification on the LightCycler™ with hybridization probes," Biochemica, 2:12-15. In other embodiments, the probe is labeled with a single chromophore that interacts with a second chromophore either conjugated with or intercalated into the target nucleic acid. See U.S. Patent No. 5,871,908.

Alternatively, in order to achieve an even higher degree of multiplexing, two or more reporter moieties or set of reporter moieties can be used. For example, a first reporter moiety is used with the first two primers, each primer having a different annealing temperature but detected with the same type of detectable signal, and a second reporter moiety can be used with an additional two primers, each primer having the same annealing temperatures as the first two primers, but because they are labeled with a second type of detectable signal, they are separately detectable from the first two primers. For example, referring to an extension of the experiment depicted in Figs. 1A-1D, each of Primer/Probe Sets A and B use the same first reporter moiety, and an additional pair of Primer/Probe sets, e.g., Sets C and D (not shown), include a second reporter moiety, wherein the first and second reporter moieties each generate a different detectable signal. Therefore, each of the individual target sequences can be detected using annealing temperature and/or reporter moiety. In this example, A is distinguished from B because they anneal to the target at different temperatures even though they are labeled with the same reporter moiety; but A is distinguished from C and D at least because amplification of A is detected using a different reporter moiety than that observed during amplification of C and D. In the examples described hereinabove and illustrated in the figures, a two-set primer annealing/elongation step is used, but it will be understood by the skilled artisan that if additional primer/probe sets are used and higher levels of multiplexing are desired, additional primer/annealing/elongation steps can be included in the method.

The selection of the appropriate temperatures for the primer annealing/elongation steps is dependent on the selected thermostable polymerase used. In the none limiting examples provided herein, the denaturation step is conducted at a temperature between 94-98°C for 20-30 seconds, e.g., at 95°C; the probe annealing step is done at 70-75°C for at least 10 seconds, e.g., 70°C; the first primer annealing/extension step is done at 62-67°C for at least 10 seconds, e.g., 62°C; and the second primer annealing/extension step is done at 50-55°C for at least 10 seconds, e.g., 50°C. In an alternative embodiment, the denaturation step is conducted at a temperature of between 94-98°C for 20-30 seconds, e.g., at 95°C; the probe annealing step is done at 70-75°C for at least 10 seconds, e.g., 72°C; the first primer annealing/extension step is done at 62-67°C for at least 10 seconds, e.g., 67°C; and the second primer annealing/extension step is done at 60-65°C for at least 10 seconds, e.g., 62°C. Each of the annealing/extension steps can be held at a constant temperature for at least 20 seconds, e.g., at least 15 seconds, between 5-10 seconds, and in one embodiment, at least 10 seconds.

Various thermostable nucleic acid polymerases are known in the art that can be selected for use in the methods described herein. In one embodiment, a Z05 polymerase or mutant thereof is used. Alternatively, an rTth polymerase or a mutant thereof is used. Sometimes it is advantageous to use a polymerase lacking the 5'-3' nuclease activity. It is sometimes desirable to use a polymerase without the proof-reading (3'-5'-exonuclease) activity. Moreover, a mixture of polymerases can be used, e.g., a first polymerase suitable for amplification of a first primer/ probe set, and a second polymerase suitable for amplification of a second primer/probe set. The target nucleic acid sequence detected by the method of the present disclosure can be of any length. Typically, the target nucleic acid is between 100 and 1,000 nucleotides in length. However, longer (several thousand nucleotides) and shorter (between 50 and 100 nucleotides) target sequences may also be used in some embodiments of the present disclosure. A target nucleic acid sequence may be contained within a larger nucleic acid molecule, isolated from a natural or laboratory-derived sample source.

While this disclosure generally refers to a method in which multiple targets are present in the sample, it will be appreciated that in some embodiments there is only one target sequence present in a sample. In a typical embodiment of the present disclosure, a "multiplex" reaction is performed where at least two and up to twelve or more different target sub-sequences or loci are detected. These embodiments generally, but not always, involve the use of a separate amplification primer and a separate probe for each target sub-sequence or loci. However, in some embodiments, the same nucleic acid, which is amplified using the same primer, may be detected with more than one probe. This is advantageous where a single sequence contains several targets or loci of interest, for example, several potential mutation sites. Each primer/probe set will be able to detect the mutation at each site.

The amplification primers of the present disclosure are oligonucleotides at least partially complementary to at least one of the existing variants of the target sequence. The length of the primer may range between 6 and 100 nucleotides, although most primers typically range between 15 and 35 nucleotides. The methods of optimizing the primers for nucleic acid amplification have been described for example, in PCR Protocols: A Guide to Methods and Applications, Innis et al., eds., (1990) Academic Press. Typically, primers are synthetic oligonucleotides, composed of A, C, G and T nucleotides. However, unconventional base nucleotides that can be incorporated into nucleic acids can also be used in primers. For example, certain modified bases are known to increase specificity of amplification, see U.S. Patent No. 6,001,011.

The design of hybridization probes is known in the art. In some embodiments of the present disclosure, more than one probe may be present in the reaction mixture subjected to an assay. One of skill in the art would immediately recognize the design criteria applicable to probes useful in multiplex assays. Specifically, the probes capable of being used in the same reaction mixture should be designed to have a distinct hybrid annealing temperature with their corresponding target sequences.

### Sample Processing Device

The methods described herein can be implemented in a sample processing device configured to perform a nucleic acid amplification technique.

In one embodiment, the methods disclosed herein are implemented in a device comprising self-contained microscale to macroscale channels, chambers, reservoirs, detection and processing regions. The device can be a cartridge, device, container, or pouch, e.g., as described in U.S. Patent Nos. 6,440,725; 6,783,934; 6,818,185; 6,979,424; 8,580,559; and 8,940,526, as well as devices such as those available from Cepheid Corp., Idaho Technology, Inc., and/or Biofire Diagnostics, Inc.

For example, the methods described herein can be implemented in a self-contained nucleic acid analysis pouch which includes a cell lysis zone, a nucleic acid preparation zone, a first-stage amplification zone, a second-stage amplification zone, as shown in Fig. 1 of US Application Publication No. 201000056383. The pouch comprises a variety of channels and blisters of various sizes and is arranged such that the sample flows through the system and various zones and processed accordingly. Sample processing occurs in various blisters located within the pouch. Numerous channels are provided to move the sample within and between processing zones, while other channels are provided to deliver fluids and reagents to the sample or to remove such fluids and reagents from the sample. Liquid within the pouch is moved between blisters by pressure, e.g., pneumatic pressure.

In an alternative example, the methods described herein can be implemented in a self-contained nucleic acid analysis cartridge as shown in Figs. 3-5 and 9 of U.S. Patent No. 9,322,052. The cartridge includes, *inter alia,* multiple chambers comprising a sample chamber for holding a fluid sample introduced through the inlet port, a wash chamber for holding a wash solution, a reagent chamber for holding a lysing reagent, a lysis chamber, a waste chamber for receiving used sample and wash solution, a neutralizer chamber for holding a neutralizer, and a master mix chamber for holding a master mix (e.g., amplification reagents and fluorescent probes) and for mixing the reagents and probes with analyte separated from the fluid sample, a reaction vessel, and a detection chamber.

In a specific embodiment, the methods described herein are conducted in a sample processing device such as that described in U.S. Patent No. 7,718,421. Segmented devices, such as those described in U.S. Patent No. 7,718,421, provide a convenient vessel for receiving, storing, processing, and/or analyzing a biological sample. In certain embodiments, the segmented device facilitates sample processing protocols involving multiple processing steps. In certain embodiments, a sample may be collected in a sample device, and the device is then positioned in an analyzer which manipulates the device and its contents to process the sample.

A particular embodiment includes a flexible device which has been segmented into compartments by breakable seals. The individual segments may contain various reagents and buffers for processing a sample. Clamps and actuators may be applied to the device in various combinations and with various timings to direct the movement of fluid and to cause the breakable seals to burst. This bursting of the breakable seals may leave an inner device surface that is substantially free of obstructions to fluid flow. In one embodiment, the flow of the biological sample may be directed toward the distal end of the device as the processing progresses, while the flow of waste may be forced to move in the opposite direction, toward the opening of the device where the sample was initially input. This sample inlet can be sealed, possibly permanently, by a cap with a locking mechanism, and a waste chamber may be located in the cap to receive the waste for storage. A significant benefit of this approach is that the processed sample does not come into contact with surfaces that have been touched by the unprocessed sample. Consequently, trace amounts of reaction inhibitors present in the unprocessed sample that might coat the walls of the device are less likely to contaminate the processed sample.

The sample processing device is shown in Fig. 1 of U.S. Patent No. 7,718,421 and may include a transparent flexible device capable of being configured into a plurality of segments and being substantially flattened by compression. In an embodiment, a device may have at least two segments. In an embodiment, a device may have at least three segments. The flexible device can provide operational functionality between approximately 2-105°C, compatibility with samples, targets and reagents, low gas permeability, minimal fluorescence properties, and/or resilience during repeated compression and flexure cycles. The device may be made of a variety of materials, examples of which include but are not limited to: polyolefins such as polypropylene or polyethylene, polyurethane, polyolefin co-polymers and/or other materials providing suitable characteristics.

In exemplary embodiments, one or more reagents can be stored either as dry substance and/or as liquid solutions in device segments. In embodiments where reagents may be stored in dry format, liquid solutions can be stored in adjoining segments to facilitate the reconstitution of the reagent solution. Examples of typical reagents include: lysis reagent, elution buffer, wash buffer, DNase inhibitor, RNase inhibitor, proteinase inhibitor, chelating agent, neutralizing reagent, chaotropic salt solution, detergent, surfactant, anticoagulant, germinant solution, isopropanol, ethanol solution, antibody, nucleic acid probes, peptide nucleic acid probes, and phosphothioate nucleic acid probes. In embodiments where one of the reagents is a chaotropic salt solution, a preferred component is guanidinium isocyanate or guanidinium hydrochloride or a combination thereof. In some embodiments, the order in which reagents may be stored in the device relative to the opening through which a sample is input, reflects the order in which the reagents can be used in methods utilizing the tube. In preferred embodiments, a reagent includes a substance capable of specific binding to a preselected component of a sample. For example, a substance may specifically bind to nucleic acid, or a nucleic acid probe may specifically bind to nucleic acids having particular base sequences.

In a specific embodiment, the device can also include a substrate such as a particle or a plurality of particles to facilitate the selective adsorption of nucleic acids. The particles are for example, of silica particles, magnetic particles, silica magnetic particles, glass particles, nitrocellulose colloid particles, and magnetized nitrocellulose colloid particles. In some embodiments where the particles can be paramagnetic, the particles can be captured by a magnetic field. Examples of reagents that may permit the selective adsorption of nucleic acid molecules to a functional group-coated surface are described, for example, in U.S. Pat. Nos. 5,705,628; 5,898,071; and 6,534,262. Separation can be accomplished by manipulating the ionic strength and polyalkylene glycol concentration of the solution to selectively precipitate, and reversibly adsorb, the nucleic acids to a solid phase surface. When these solid phase surfaces are paramagnetic microparticles, the magnetic particles, to which the target nucleic acid molecules have been adsorbed, can be washed under conditions that retain the nucleic acids but not other molecules. Several companies offer magnetic-based purification systems, such as QIAGEN's MagAttract™, Cortex Biochem's MagaZorb™, and Roche Life Science's MagNA Pure LC™. These products use negatively charged particles and manipulate buffer conditions to selectively bind a variety of nucleic acids to the particles, wash the particles and elute the particles in aqueous buffers. Many of the products used by these companies use chaotropic salts to aid in the precipitation of nucleic acids onto the magnetic particles. Examples are described in U.S. Pat. Nos. 4,427,580; 4,483,920; and 5,234,809.

Preferred exemplary embodiments may include a linear arrangement of 2 or more device segments (Fig. 1 of U.S. Patent No. 7,718,421). A linear arrangement facilitates moving the sample and resultant waste and target through the tube in a controlled manner. A sample, e.g., a sample, can be input through a first opening in a first segment of the device. Thereafter, waste from a processed sample can be moved back toward the first opening while the target is pushed towards the opposite end, thereby minimizing contamination of the target by reaction inhibitors that may have become attached to the device wall, and confining the target to a clean segment of the device which can contain suitable reagents for further operations of the target. Some embodiments may use a plurality of segments, each containing at least one reagent. In some embodiments, these segments may contain reagents in the following order: the second segment can include a surface comprising an immobilized binding reagent and a dilution buffer; the third segment can be partitioned into two subsections, the first including proteinase K and the second including silica magnetic beads or other suitable particle; the reagent in the fourth segment may be either a lysis reagent; the reagent in the fifth segment may be a washing buffer; the reagent in the sixth-eighth segments may be a wash buffer, a neutralization reagent, a suspension buffer, an elution reagent, or nucleic acid amplification and detection reagents. In some embodiments, the segments may be arranged continuously, while in other embodiments, these segments may be separated by another segment or segments in between.

In certain embodiments, the sequence of events in such a test may include: 1) a biological sample collected with a collection tool, 2) a flexible device, which can include a plurality of segments that may contain the reagents required during the test, and in which the collected sample can be placed using a first opening in the device, 3) at least one substrate that may be set at a controlled temperature and/or other conditions to capture target organisms or nucleic acids during a set incubation period, 4) organisms or molecules, in the unprocessed sample, that may not bind to the substrate and could thus be removed by transferring liquid to a waste reservoir, 5) storing waste, in a waste reservoir, that can be segregated from the target by a clamp and/or actuator compressed against the device, 6) a wash buffer, released from another segment of the device, that can remove reaction inhibitors, 7) an elution reagent, from another segment, that can release the target bound to the substrate after incubation at a controlled temperature, and 8) nucleic acids that can be detected by techniques well known to those familiar in the art or collected through a second opening in the device. In exemplary embodiments the flow of the sample may be from the first opening towards the distal end of the device as the test progresses while the flow of waste may be towards the closed sample input opening of the device, where a waste chamber in the cap of the device receives the waste for storage. Consequently, undesirable contact between a processed sample and surfaces in a reaction vessel that have been touched by the unprocessed sample is avoided, thereby preventing reaction inhibition due to trace amounts of reaction inhibitors present in the unprocessed sample and that might coat the walls of the reaction vessel.

Some embodiments may incorporate the use of a flexible device divided into a plurality of segments that may be transverse to the longitudinal axis of the device, and which may contain reagents; as well as an analyzer, that may have a plurality of compression members, such as actuators and/or clamps, and blocks, opposing the actuators and clamps, to process a sample. Various combinations of these actuators, clamps, and/or blocks may be used to effectively clamp the device closed thereby segregating fluid. In exemplary embodiments, at least one of the actuators or blocks may have a thermal control element to control the temperature of a device segment for sample processing. The sample processing apparatus can further have at least one magnetic field source capable of applying a magnetic field to a segment. The sample processing apparatus can further have a detection device, such as photometer or a CCD, to monitor a reaction taking place or completed within the device.

Fluid can be driven through a flow-channel by compressing the device with a centrally-positioned actuator, and its flanking clamps if any, to form a flow channel with a gap of about 1 to about 500 um, preferably about 5 to about 500 um through each segment. The adjacent actuators gently compress the adjacent segments in liquid communication with the flow-channel to generate an offset inner pressure to ensure a substantially uniform gap of the flow channel. The two flanking actuators can then alternatively compress and release pressure on the device on their respective segments to generate flow at a controlled flow rate. Optional flow, pressure, and/or force sensors may be incorporated to enable closed-loop control of the flow behavior. The flow-channel process can be used in washing, enhancing the substrate binding efficiency, and detection.

A particle immobilization and re-suspension process can be used to separate the particles from the sample liquid. The magnetic field generated by a magnetic source may be applied to a segment containing a magnetic particle suspension to capture and immobilize the particles to the tube wall. An agitation process can be used during the capturing process. In another embodiment, a flow-channel can be formed in the segment with the applied magnetic field, and magnetic particles can be captured in the flow to increase the capturing efficiency. To resuspend immobilized particles, the magnetic field may be turned off or removed, and an agitation or flow-channel process can be used for re-suspension.

A real-time detection of a signal from a device segment can be achieved by using a sensor, such as a photometer, a spectrometer, a CCD, connected to a block. In exemplary embodiments, pressure can be applied by an actuator on the device segment to suitably define the device segment's shape. The format of signal can be an intensity of a light at certain wavelength, such as a fluorescent light, a spectrum, and/or an image, such as image of cells or manmade elements such as quantum dots. For fluorescence detection, an excitation of light from the optical system can be used to illuminate a reaction, and emission light can be detected by the photometer. To detect a plurality of signals having specific wavelengths, different wavelength signals can be detected in series or parallel by dedicated detection channels or a spectrometer.

### Kits

In some embodiments, reagents, materials, and devices for carrying out the presently disclosed methods are included in a kit. In some embodiments, the kit includes components, in separate containers, vials, or compartments, including (i) a first primer/probe set including a first labeled probe and a first primer, and (ii) a second primer/probe set including a second labeled probe and a second primer, wherein the first primer anneals to a target nucleic acid of the plurality of target nucleic acids at a first temperature and the second primer anneals to an additional nucleic acid of the plurality of said target nucleic acids at a second temperature and the first temperature is higher than said second temperature. The kit can further include, in one or more separate containers, vials, or compartments, reagents necessary for amplification of target nucleic acids, including but not limited to, DNA polymerase, dNTPs, lysis and/or wash buffer, a surface, e.g., a particle, comprising an immobilized binding reagent, a dilution buffer, proteinase K, silica magnetic beads or other suitable particle, lysis reagent(S), a neutralization reagent, a suspension buffer, an elution reagent, etc., as well as reagents for obtaining, storing, and/ or preparing sample for analysis.

In addition, the kit includes an assay processing device such as that described above and in a specific embodiment, the assay processing device includes various reagents required to perform the methods disclosed herein stored within one or more segments of the device.

The kit can further include controls, e.g., a polynucleotide that is wild type at the sequence to be detected, or a polynucleotide that includes the sequence to be detected.

The kit can also include additional devices such as sample tubes or vials; reaction containers (e.g., tubes, multiwell plates, microfluidic chips or chambers, etc), as well as directions for use or reference to a website.

## Claims

1. A method for detection of a plurality of target nucleic acids in a single sample container comprising the steps of:
(a) contacting a sample suspected of containing a plurality of target nucleic acids with (i) a first primer/probe set including a first labeled probe and a first primer, and (ii) a second primer/probe set including a second labeled probe and a second primer, wherein said first primer anneals to a target nucleic acid of said plurality of target nucleic acids at a first temperature and said second primer anneals to an additional nucleic acid of said plurality of said target nucleic acids at a second temperature and said first temperature is higher than said second temperature;
(b) amplifying said plurality of target nucleic acids in the sample in an amplification reaction that includes at least a two-step annealing/elongation temperature profile including a first step comprising said first temperature and a second step comprising said second temperature;
(c) detecting, sequentially, over at least a portion of said annealing/elongation temperature profile
(i) a first dectable signal during said first step, which results from annealing/elongation of the first primer and which corresponds to the signal emitted by said first label;
(ii) a second detectable signal during said second step, which results from annealing/elongation of the first and second primers and which corresponds to the signal emitted by said first and said second label; and
(iii) subtracting said first detectable signal from said second detectable signal to identify the detectable signal emitted by said second label; and
(d) measuring a relative amount of each target nucleic acid in said plurality of target nucleic acids.

2. The method of claim 1, wherein said first and second labeled probes are each labeled with the same reporter moiety.

3. The method of claim 2, wherein said reporter moiety is fluorescent.

4. The method of any one of claims 1 to 3, wherein said first and second labeled probes are each labeled with a reporter moiety and a quencher moiety.

5. The method of claim 4, wherein said reporter moiety and said quencher moiety are fluorophores.

6. The method of any one of claims 4 to 5, wherein said reporter moiety is a fluorophore and said quencher moiety is a dark quencher.

7. The method of any one of claims 1 to 6, wherein said first and second steps, respectively, are held for at least 10 seconds.

8. The method of any one of claims 1 to 7, wherein said amplification reaction is performed in the presence of a mixture of DNA polymerases.

9. The method of any one of claims 1 to 8, wherein the first and second temperatures are at least 10 degrees apart.

10. The method of claim 9, wherein the first and second temperatures are at least 15 degrees apart.

## Patentansprüche

1. Verfahren zum Nachweisen einer Vielzahl von Zielnukleinsäuren in einem einzelnen Probenbehälter, umfassend die folgenden Schritte:
(a) Inkontaktbringen einer Probe, die vermutlich eine Vielzahl von Zielnukleinsäuren enthält, mit (i) einem ersten Primer/Sonde-Satz, der eine erste markierte Sonde und einen ersten Primer einschließt, und (ii) einem zweiten Primer/Sonde-Satz, der eine zweite markierte Sonde und einen zweiten Primer einschließt, wobei der erste Primer bei einer ersten Temperatur an eine Zielnukleinsäure der Vielzahl von Zielnukleinsäuren annealt und der zweite Primer bei einer zweiten Temperatur an eine zusätzliche Nukleinsäure der Vielzahl von Zielnukleinsäuren annealt und die erste Temperatur höher ist als die zweite Temperatur;
(b) Amplifizieren der Vielzahl von Zielnukleinsäuren in der Probe in einer Amplifikationsreaktion, die mindestens ein Zwei-Schritt-Annealing/Verlängerung-Temperaturprofil einschließt, das einen ersten Schritt, umfassend die erste Temperatur, und einen zweiten Schritt, umfassend die zweite Temperatur, einschließt;
(c) sequentielles Nachweisen über mindestens einen Abschnitt des Annealing/Verlängerung-Temperaturprofils
(i) eines ersten nachweisbaren Signals während des ersten Schrittes, das sich aus dem Annealing/der Verlängerung des ersten Primers ergibt und das dem von der ersten Markierung ausgesendeten Signal entspricht;
(ii) eines zweiten nachweisbaren Signals während des zweiten Schrittes, das sich aus dem Annealing/der Verlängerung des ersten und zweiten Primers ergibt und das dem von der ersten und der zweiten Markierung ausgesendeten Signal entspricht; und
(iii) Subtrahieren des ersten nachweisbaren Signals von dem zweiten nachweisbaren Signal, um das von der zweiten Markierung ausgesendete nachweisbare Signal zu identifizieren; und
(d) Messen einer relativen Menge jeder Zielnukleinsäure in der Vielzahl von Zielnukleinsäuren.

2. Verfahren nach Anspruch 1, wobei die erste und zweite markierte Sonde jeweils mit derselben Reportereinheit markiert sind.

3. Verfahren nach Anspruch 2, wobei die Reportereinheit fluoreszierend ist.

4. Verfahren nach einem der Ansprüche 1 bis 3, wobei die erste und zweite markierte Sonde jeweils mit einer Reportereinheit und einer Quencher-Einheit markiert sind.

5. Verfahren nach Anspruch 4, wobei die Reportereinheit und die Quencher-Einheit Fluorophore sind.

6. Verfahren nach einem der Ansprüche 4 bis 5, wobei die Reportereinheit ein Fluorophor ist und die Quencher-Einheit ein Dunkel-Quencher ist.

7. Verfahren nach einem der Ansprüche 1 bis 6, wobei der erste bzw. der zweite Schritt für mindestens 10 Sekunden gehalten werden.

8. Verfahren nach einem der Ansprüche 1 bis 7, wobei die Amplifikationsreaktion in der Gegenwart eines Gemisches aus DNA-Polymerasen durchgeführt wird.

9. Verfahren nach einem der Ansprüche 1 bis 8, wobei die erste und die zweite Temperatur mindestens 10 Grad auseinanderliegen.

10. Verfahren nach Anspruch 9, wobei die erste und die zweite Temperatur mindestens 15 Grad auseinanderliegen.

## Revendications

1. Procédé de détection d'une pluralité d'acides nucléiques cibles dans un récipient d'échantillon unique comprenant les étapes de :
(a) mise en contact d'un échantillon soupçonné de contenir une pluralité d'acides nucléiques cibles avec (i) un premier ensemble amorce/sonde incluant une première sonde marquée et une première amorce, et (ii) un second ensemble amorce/sonde incluant une seconde sonde marquée et une seconde amorce, dans lequel ladite première amorce s'hybride à un acide nucléique cible de ladite pluralité d'acides nucléiques cibles à une première température et ladite seconde amorce s'hybride à un acide nucléique supplémentaire de ladite pluralité desdits acides nucléiques cibles à une seconde température et ladite première température est supérieure à ladite seconde température ;
(b) amplification de ladite pluralité d'acides nucléiques cibles dans l'échantillon dans une réaction d'amplification qui inclut au moins un profil de température d'hybridation/allongement en deux étapes incluant une première étape comprenant ladite première température et une seconde étape comprenant ladite seconde température ;
(c) détection, séquentiellement, sur au moins une partie dudit profil de température d'hybridation/allongement
(i) d'un premier signal détectable pendant ladite première étape, qui résulte de l'hybridation/allongement de la première amorce et qui correspond au signal émis par ledit premier marqueur ;
(ii) d'un second signal détectable pendant ladite seconde étape, qui résulte de l'hybridation/allongement des première et seconde amorces et qui correspond au signal émis par ledit premier et ledit second marqueur ; et
(iii) soustraction dudit premier signal détectable dudit second signal détectable pour identifier le signal détectable émis par ledit second marqueur ; et
(d) mesure d'une quantité relative de chaque acide nucléique cible dans ladite pluralité d'acides nucléiques cibles.

2. Procédé selon la revendication 1, dans lequel lesdites première et seconde sondes marquées sont chacune marquées avec la même fraction rapporteur.

3. Procédé selon la revendication 2, dans lequel ladite fraction rapporteur est fluorescente.

4. Procédé selon l'une quelconque des revendications 1 à 3, dans lequel lesdites première et seconde sondes marquées sont chacune marquées avec une fraction rapporteur et une fraction extincteur.

5. Procédé selon la revendication 4, dans lequel ladite fraction rapporteur et ladite fraction extincteur sont des fluorophores.

6. Procédé selon l'une quelconque des revendications 4 à 5, dans lequel ladite fraction rapporteur est un fluorophore et ladite fraction extincteur est un extincteur sombre.

7. Procédé selon l'une quelconque des revendications 1 à 6, dans lequel lesdites première et seconde étapes, respectivement, sont maintenues pendant au moins 10 secondes.

8. Procédé selon l'une quelconque des revendications 1 à 7, dans lequel ladite réaction d'amplification est mise en œuvre en présence d'un mélange d'ADN polymérases.

9. Procédé selon l'une quelconque des revendications 1 à 8, dans lequel les première et seconde températures sont séparées d'au moins 10 degrés.

10. Procédé selon la revendication 9, dans lequel les première et seconde températures sont séparées d'au moins 15 degrés.
